# EUROPEAN PATENT SPECIFICATION

(11) **EP 0 944 351 B1**
(45) Date of publication and mention of the grant of the patent: **23.03.2005**
(21) Application number: 97942675.6
(22) Date of filing: 24.09.1997
(51) Int. Cl.: A61B 5/028, G01F 1/68, G01F 1/684

(54) **REDUCED-NOISE CATHETER**
KATHETER MIT VERMINDERTEM RAUSCHEN
CATHETER A BRUIT REDUIT

(30) Priority: 02.10.1996 US 725117
(43) Date of publication of application: 29.09.1999
(73) Proprietor: Edwards Lifesciences Corporation, Irvine, CA 92614 (US)
(72) Inventor: MOONEY, Charles, R., Costa Mesa, CA 92626 (US); KONNO, Mark, A., Laguna Beach, CA 92651 (US); MINOT, Mark, M., Aliso Viejo, CA 92656 (US); PHAN, Luong, N., Laguna Niguel, CA 92677 (US); ROTELIUK, Luchy, D., Lake Forest, CA 92630 (US)
(74) Representative: Baker, Colin John
(86) International application number: PCT/US1997/017064
(87) International publication number: WO 1998/014114

(56) References cited:
- EP-A- 0 440 155
- US-A- 3 359 974
- US-A- 4 240 441

## Description

### FIELD OF THE INVENTION

The present invention relates to catheters for measuring cardiac functions and, more particularly, to catheters which incorporate a temperature sensor for measuring cardiac output.

### BACKGROUND OF THE INVENTION

Cardiac functions such as intercardiac pressure, flow rate, and ejection fraction of a patient are monitored by physicians in order to obtain vital information necessary to perform various cardiovascular procedures. Of the various cardiac functions, cardiac output provides a physician with the amount of blood discharged from one ventricle of the heart, typically the right ventricle, and flowing through the cardiovascular system at a given time.

Cardiac output is preferably measured continuously throughout a particular surgical procedure. The cardiac output of a patient may also be measured before and after a surgical procedure. To measure cardiac output, the physician inserts a catheter into the cardiovascular system and positions the catheter at a target site. The catheter has a heating element which is activated to heat the blood flowing around the catheter at the target sight. A temperature sensor, for example, a thermistor, positioned downstream of the heating element provides a signal from which blood temperature is calculated. Cardiac output may then be calculated based upon the blood temperature in conjunction with other variables. One such device for the thermal determination of cardiac performance is described in US Patent 3,359,974.

The signal from the temperature sensor is relatively small. Accordingly, conventional temperature-sensing catheters are vulnerable to a number of error-inducing sources. A surgical theater is a particularly noisy electrical environment, due in part to multiple machines operating simultaneously. The human body is also very noisy. All of this electronic, thermal, and physiological noise decreases the accuracy of the signal from the temperature sensor. Although sophisticated signal processing techniques and equipment have been employed in the prior art to reduce the amount of noise (and resultant error) as much as possible, there still exists a significant amount of noise in the measurement of cardiac output. These noise problems affect the accuracy of cardiac output measurements and hinder a physician's ability to successfully diagnose, monitor, and treat cardiovascular problems of a patient.

### SUMMARY OF THE INVENTION

Accordingly, it is an object of the present invention to provide a temperature-sensing catheter which mitigates and/or obviates the aforementioned drawbacks of conventional temperature-sensing catheters.

It is another object of the invention to provide a temperature-sensing catheter which measures cardiac output with a significant improvement in accuracy over conventional catheters.

It is yet another object of the present invention to provide a temperature-sensing catheter having significantly reduced noise resulting from heating element operation.

These objects, as well as other objects, features, and advantages of the present invention, are achieved by providing a catheter in which temperature-sensor wires connected to a temperature sensor are twisted. The inventors of the present invention have discovered that errant noise in a voltage signal carried on the temperature-sensor wires from the temperature sensor results from an induced bias voltage. This errant bias voltage is caused by thermal, electromagnetic, or combined thermal and electromagnetic effects.

According to one aspect of the present invention, a catheter includes a body portion having a distal end and a proximal end. A plurality of lumens are formed in the body portion between the distal and proximal ends. A heating element and a temperature sensor, preferably a thermistor, are disposed on the catheter, with the temperature sensor positioned between the heating element and the distal end of the body portion. Heating-element wires are connected to the heating element and extend from the heating element to the proximal end of the body portion in one of the lumens of the catheter, and temperature-sensor wires are connected to the temperature sensor and extend in a twisted configuration from the temperature sensor to the proximal end of the body portion in one of the lumens of the catheter. The heating-element wires are connectable to a control unit and carry an activation signal from the control unit to the heating element to activate the heating element. The temperature-sensor wires are connectable to a processing unit and carry a temperature-sensor signal from the temperature sensor to the processing unit for processing.

An advantage of the catheter of the present invention over conventional catheters is that the twisted configuration of the temperature-sensor wires significantly reduces the amount of noise and, accordingly, the amount of error in measuring cardiac output. The inventors of the present invention have determined that a flow differential of cardiac output, which is the difference between the actual blood flow and the measured blood flow, may be reduced by about 80%. This significant reduction in flow differential provides a greatly improved, highly accurate temperature-sensing catheter for measuring cardiac output.

One of the features of the invention is that the temperature-sensor wires may be received in the same lumen within which the heating-element wires are received. Prior to the present invention, heating-element wires induced a significant cross-talk signal onto the temperature-sensor wires. Accordingly, attempts were made to isolate and shield the temperature-sensor wires in another lumen. However, the twisted configuration of the temperature-sensor wires substantially eliminates cross talk from the heating-element wires. Accordingly, the wires may be received within the same catheter. This allows one additional lumen to be used for another purpose, thereby increasing the functionality of the catheter. Alternatively, the catheter may be made with a reduced outer diameter, thereby providing reduced risk of trauma to the cardiovascular system of the patient.

Other aspects, features, and advantages of the present invention will become apparent to those skilled in the art from a reading of the following detailed description with reference to the accompanying drawings.

### BRIEF DESCRIPTION OF THE DRAWINGS

FIGURE 1 is a perspective view of a reduced-noise catheter shown in a preferred embodiment of the present invention;
FIGURE 2A is a cross-sectional view of the reduced-noise catheter of the invention, taken along lines 2-2 of FIGURE 1, particularly illustrating a preferred embodiment of the catheter in which heating-element wires and temperature-sensor wires are positioned within separate lumens of the catheter;
FIGURE 2B is cross-section view similar to that of FIGURE 2A, particularly illustrating another preferred embodiment of the catheter in which the heating-element wires and the temperature-sensor wires are positioned within the same lumen of the catheter;
FIGURE 3 is a cross-sectional view of the reduced-noise catheter of the invention, taken along line 3-3 of FIGURE 1, particularly illustrating a section of the catheter at which a heating element is disposed;
FIGURE 4 is a developmental view of a preferred embodiment of a heating element of the reduced-noise catheter of the present invention;
FIGURE 5 is a cross-sectional view of the reduced-noise catheter of the invention, taken along line 5-5 of FIGURE 1, particularly illustrating a section of the catheter at which a temperature sensor is disposed;
FIGURE 6A is a perspective view of a pair of separate temperature-sensor wires shown in a substantially parallel condition;
FIGURE 6B is a perspective view of a pair of separate temperature-sensor wires for a reduced-noise catheter in accordance with the present invention, illustrating the pair of separate temperature-sensor wires in a braided or twisted condition;
FIGURE 7A is a perspective view of bifilar temperature-sensor wires shown in an untwisted condition;
FIGURE 7B is a perspective view of bifilar temperature-sensor wires for a reduced-noise catheter in accordance with the present invention, illustrating the bifilar temperature-sensor wires in a twisted condition.;
FIGURE 8 is a schematic representation of a preferred implementation of the reduced-noise catheter of the present invention, illustrating the measurement of cardiac output;
FIGURE 9 is a graph illustrating flow differentials of conventional catheters and preferred embodiments of the reduce-noise catheter of the present invention;
FIGURE 10A is a schematic representation of a circuit including a thermistor and a processing unit for measuring cardiac output;
FIGURE 10B is a schematic representation of a circuit including a thermistor and a processing unit for measuring cardiac output configured as a voltage divider;
FIGURE 10C is a schematic representation of a circuit including a thermistor and a processing unit for measuring cardiac output configured as a current divider; and
Figure 11 is-a graph illustrating voltage measurements as a function of a change in temperature of the circuits represented, in FIGURES 10A and 10B.

### DETAILED DESCRIPTION OF THE PREFERRED EMBODIMENT

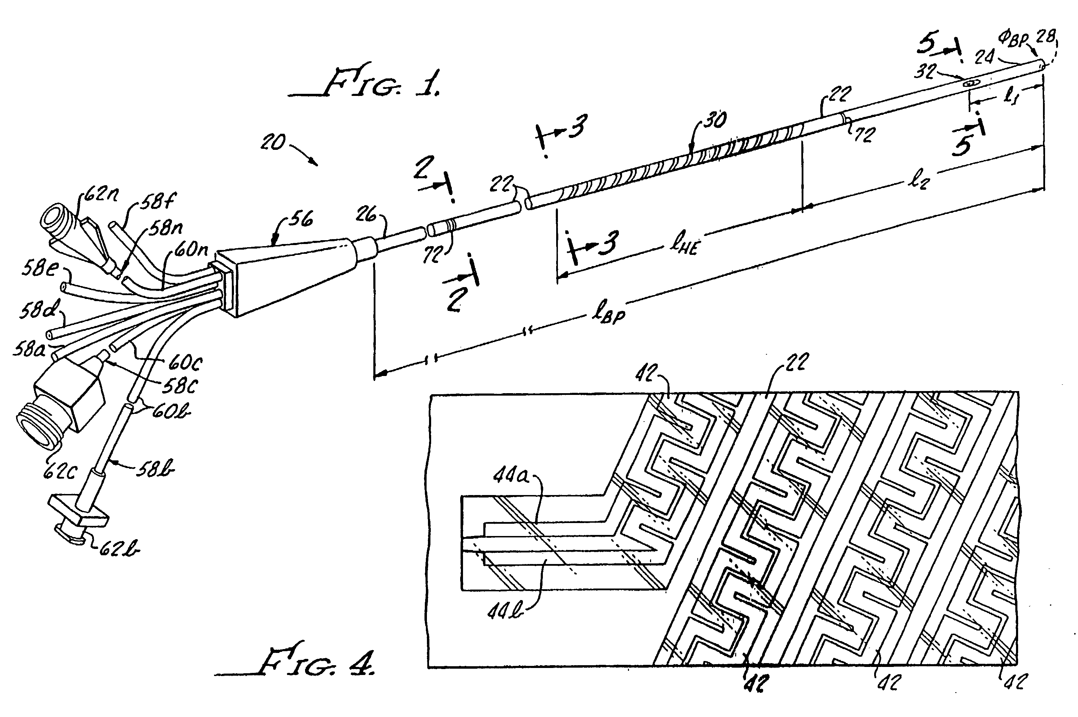

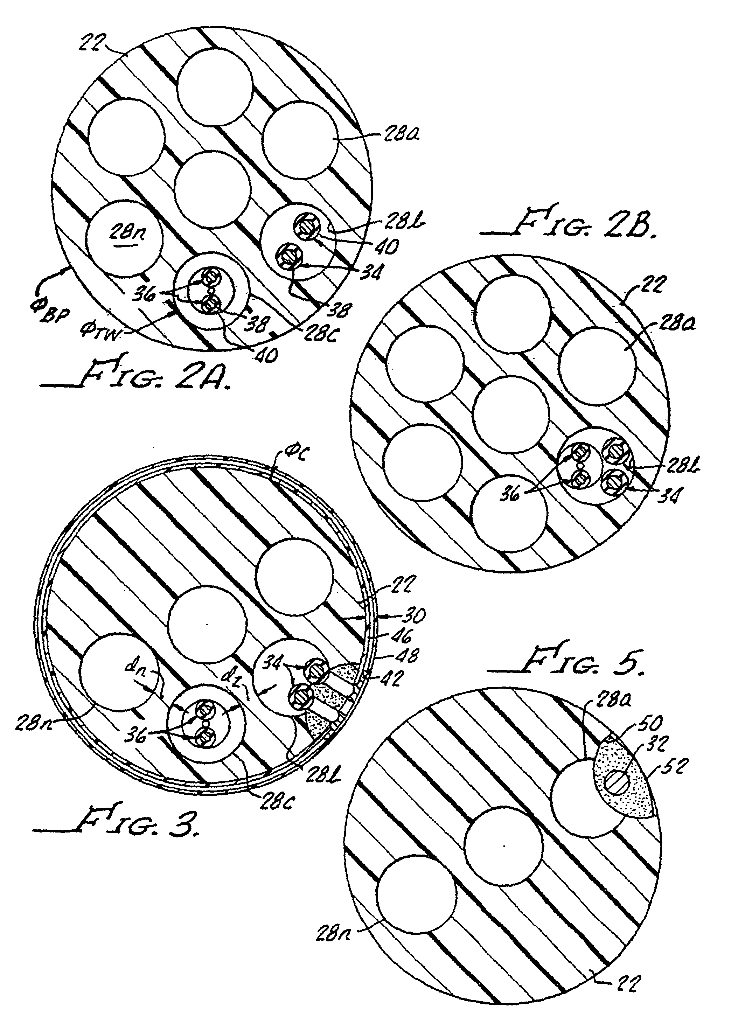

Referring to the drawings, particularly to FIGURES 1 and 2A, a reduced-noise catheter 20 is illustrated in accordance with a preferred embodiment of the present invention. The reduced-noise catheter 20 generally includes a body portion 22 which has a distal end 24 and a proximal end 26. At least one but preferably a plurality of lumens are formed in the body portion 22 longitudinally between the distal end 24 and the proximal end 26 thereof. The lumens are generally indicated by reference numeral 28 and specifically indicated by reference numeral 28 with an alpha suffix, i.e., 28a, 28b, 28c, ... 28n, which is shown in FIGURE 2A. This alphanumeric numbering convention will be followed as appropriated herein.

A heating element 30 is disposed between the distal end 24 and the proximal end 26 of the body portion 22, and a temperature sensor 32, preferably a thermistor, is disposed between the heating element 30 and the distal end 24 of the body portion 22. Heating-element wires 34 are connected to the heating element 30 and extend from the heating element at least to the proximal end 26 of the body portion 22 within one of the lumens 28. Temperature-sensor wires 36 are connected to the temperature sensor 32 and also extend at least to the proximal end 26 of the body portion 22 in one of the lumens 28.

Specifically referencing FIGURE 2A, the heating-element wires 34 and the temperature-sensor wires 36 are shown to occupy separate lumens 28, specifically lumen 28b and lumen 28c, respectively. However, according to a preferred embodiment of the invention illustrated in FIGURE 2B, the heating-element wires 34 and the temperature-sensor wires 36 are positioned within the same lumen 28, specifically lumen 28b. The preferred embodiments of the reduced-noise catheter 20 shown in FIGURES 2A and 2B, particularly the novel features of the temperature-sensor wires 36, will be discussed in more detail below. In addition, the respective configurations and functions of the heating-element wires 34 and the temperature-sensor wires 36, each wire of which may include a conductor 38 and an insulator 40, will also be discussed in more detail below.

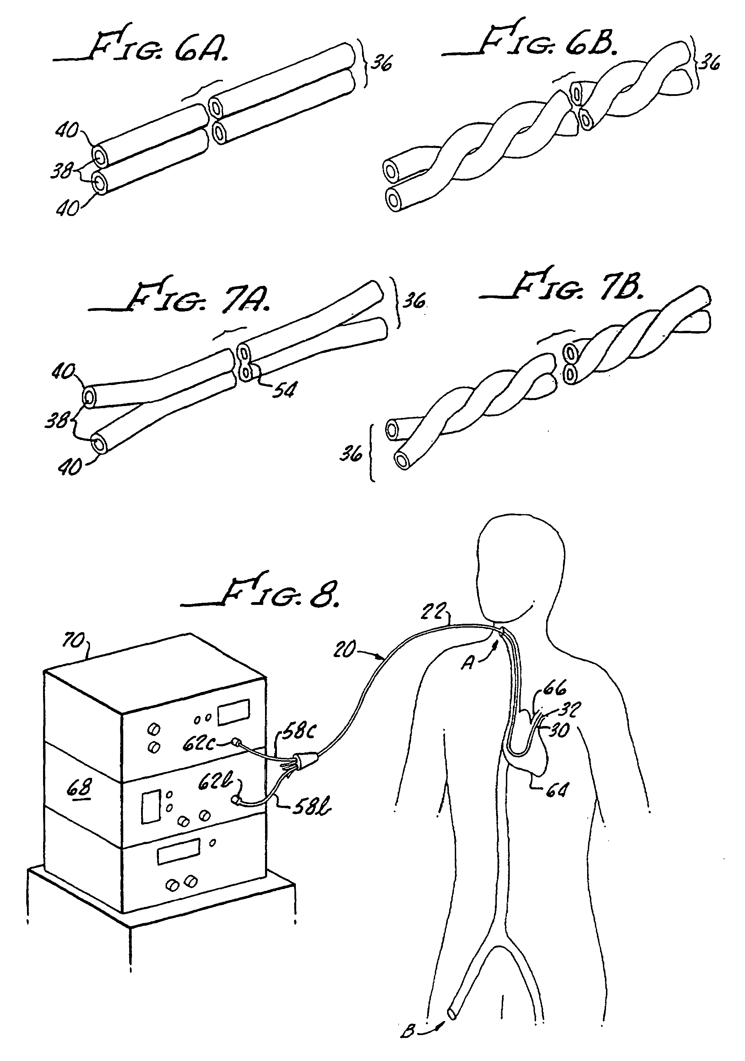

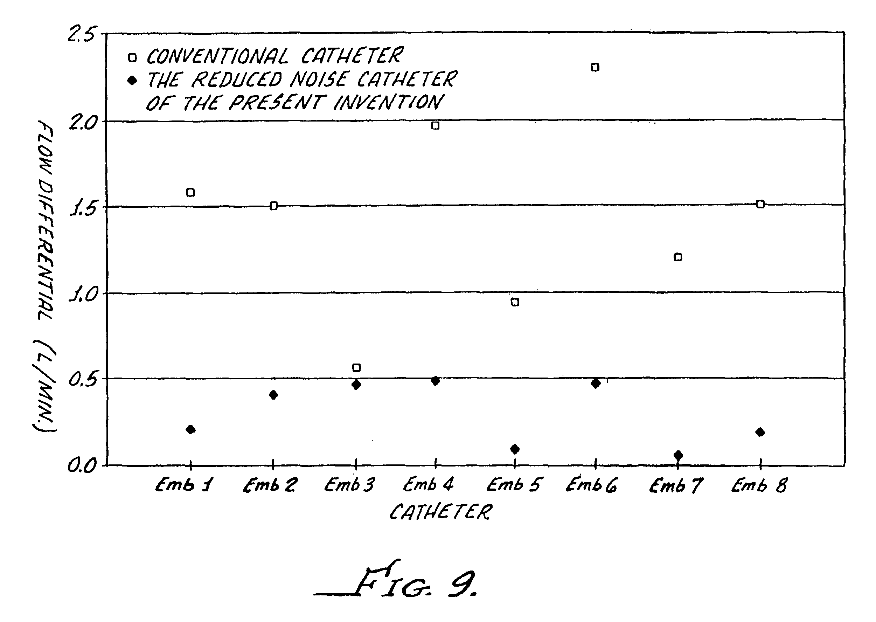

With reference to FIGURES 3 and 4, a preferred configuration of the heating element 30 of the reduced-noise catheter 20 is shown. The heating element 30 is preferably in the form of a conductive ribbon-like filament 42 spirally wrapped around an external surface and along a length l_{HE} of the body portion 22. The filament 42 is configured such that an overall length of the filament itself, if laid straight from end to end, is substantially longer than length l_{HE} of the body portion 22. Accordingly, the relationship between the resistance and the surface area of the filament 42 allows an efficient radiation of heat from the filament 42 when activated by a power supply. Further, the filament 42 may be thought to have a predetermined number of turns or revolutions around the external surface of the body portion 22, such that the pitch of the heating element 30 may be determined by dividing the predetermined number of revolutions of the filament 42 by length l_{HE}. Although a specific configuration of a preferred embodiment of the heating element 30 is shown in FIGURES 3 and 4, there are numerous commercially available heating elements and modifications of the configuration illustrated in the drawings which may be suitably used in alternative embodiments of the invention.

At each end of the filament 42 formed a lead 44 (specifically 44a and 44b) to which the heating-element wires 34 are respectively connected. In order to connect the heating-element wires 34 to the leads 44 of the filament 42, an aperture (not shown) is formed through the body portion 22-between the external surface of the body portion 22 and the lumen 28 in which the heating-element wires 34 are positioned (i.e., lumen 28b)-so that the heating-element wires 34 are accessible to the leads 44.

An insulating strip 46 may be positioned between the filament 42 and the external surface of the body portion 22. The configuration of the insulating strip 46 preferably corresponds to that of the filament 42. A protective outer coating 48 may also be applied around the section of the body portion 22 at which the filament 42 is positioned.

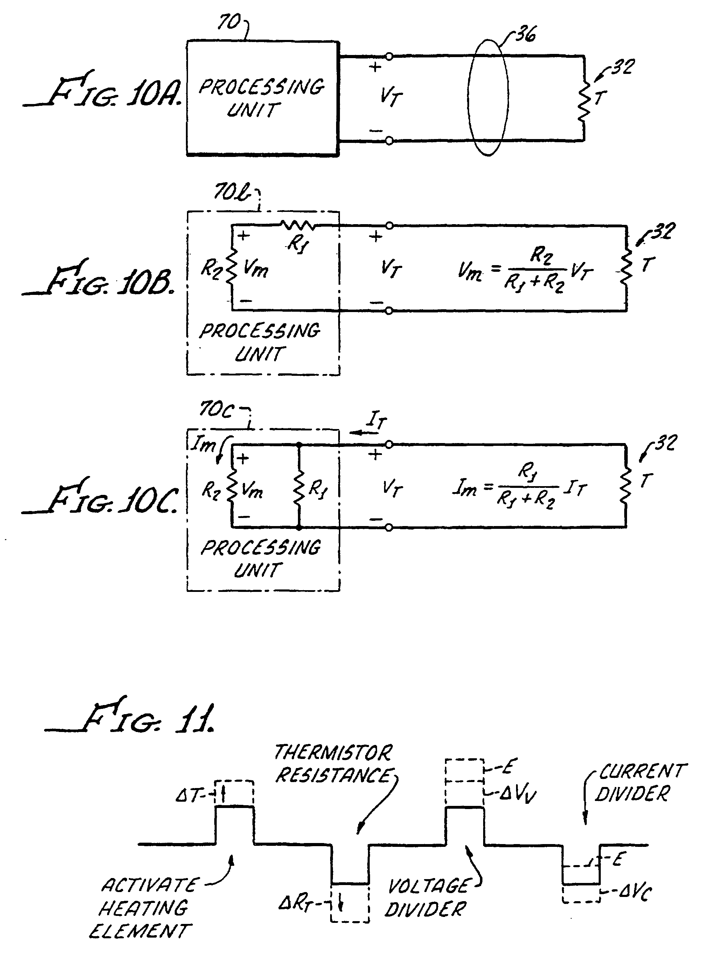

With reference to FIGURE 5, a preferred embodiment of the temperature sensor 32 of the reduced-noise catheter 20 is shown. The temperature sensor 32 is received in a port 50 which is formed in the body portion 22, extending from the external surface of the body portion 22 to the lumen 28 in which the temperature-sensor wires 36 are positioned (i.e., lumen 28c). The temperature sensor 32 has a pair of insulated leads (not shown) which are accessible to the temperature-sensor wires 36 through the port 50 and to which the temperature-sensor wires 36 are respectively connected. A protective coating 52 is preferably applied over the temperature sensor 32 within the port 50.

As previously mentioned, the temperature-sensor wires 36 of the reduced-noised catheter 20 of the present invention are twisted. For example, the temperature-sensor wires 36 are initially separate wires, as shown in FIGURE 6A, which are brought together so that the insulating coatings 40 of the conductors 38 contact. The temperature-sensor wires 36 are then twisted together, as shown in FIGURE 6B. Alternatively, referencing FIGURE 7A, the temperature-sensor wires 36 may be bifilar which means that the insulating coatings 40 of the conductors 38 are attached along a seam 54. The bifilar temperature-sensor wires 36 are then twisted, resulting in the configuration shown in FIGURE 7B. The pitch of the temperature-sensor wires 36 shown in FIGURES 6B and 7B may vary according to the particular embodiment of the reduced-noise catheter 20. However, it is preferable for the pitch of the temperature-sensor wires 36 to be at least about two revolutions per centimeter (cm) (or about five revolutions per inch) and preferably within the range of about two revolutions per cm to about six revolutions per cm (or about 15 revolutions per inch).

According to a preferred embodiment of the reduced-noise catheter 20 of the present invention illustrated in FIGURE 1, a coupling apparatus 56 may be connected to the proximal end 26 of the body portion 22. The coupling apparatus 56 includes a plurality of lumens (not shown) corresponding to and in communication with the lumens 28 of the body portion 22. From the lumens of the coupling apparatus 56 extends a plurality of coupling units 58. Each of the coupling units 58a, 58b, 58c, ... 58n includes a lead 60 and a couple 62 which are configured for the measurement of a specific cardiovascular function. For example, any number of the couple units 58 may be used in the electrical measurement of various cardiac functions, in the introduction of fluids into the vascular system, or in conjunction with a balloon disposed at the distal end 24 of the body portion 22, and so on as known in the art.

One of the coupling units 58 is used in conjunction with the heating element 30 (for example, coupling unit 58b), and another one of the coupling units is used in conjunction with the temperature sensor 32 (for example, coupling unit 58c). Accordingly, the heating-element wires 34 extend from lumen 28b of the body portion 22, through a corresponding lumen of the coupling apparatus 56, and through lead 60b to couple 62b of coupling unit 58b. Similarly, the temperature-sensor wires 36 extend from lumen 28c of the body portion 22, through a corresponding lumen of the coupling apparatus 56, through lead 60c to couple 62c of coupling unit 58c.

A preferred implementation of the reduced-noise catheter 20 of the present invention is illustrated in FIGURE 8. The body portion 22 of the catheter 20 may be inserted into the cardiovascular system of a patient, which includes a heart 64 and blood vessels, by making an incision in the patient and inserting the body portion 22 into a blood vessel of the patient. The body portion 22 is urged intravascularly until the distal end 24 is positioned at a target location with the temperature sensor 32 downstream of the heating element 30. The target location is preferably within the pulmonary artery 66 so that the cardiac output of the right ventricle of the heart 64 may be measured.

The body portion 22 of the catheter 20 may be inserted either jugularly, as indicated by A in FIGURE 8, or femorally, as indicated by B in FIGURE 8. Jugular access leads the catheter 20 through the superior vena cava and into the right atrium of the heart 64. Femoral access leads the catheter 20 into the inferior vena cava and into the right atrium of the heart 64. Once in the right atrium, the distal end 24 may be urged into the right ventricle and through the pulmonary valve into the pulmonary artery 66.

With the body portion 22 positioned in the patient, the couple 62 corresponding to the heating element 30 (i.e., couple 62b) is connected to a control unit 68 for providing electrical signals (for example, a cycle of pseudorandom binary sequences) to activate the heating element 30, and the couple 62 corresponding to the temperature sensor 32 (i.e., couple 62c) is connected to a processing unit 70 for processing electrical signals from the temperature sensor 32. The electrical signals from the temperature sensor 32 are used to calculate blood temperature which, in turn, is used to calculate cardiac output or blood flow.

Generally speaking, in operation the heating element 30 is activated, heating the volume of blood passing over and around the heating element 30. The resistivity of the temperature sensor 32 changes from the heating of the blood, changing the signal received at the processing unit 70. Based upon known variables, cardiac output may be calculated. These calculations are known in the art and will not be detailed herein. In the preferred embodiment of the catheter 20 in which the temperature sensor 32 is a thermistor, the resistivity of the thermistor decreases as the temperature of the blood increases.

In order to substantially eliminate a DC bias signal in the electrical signals from the temperature sensor 30, the temperature-sensor wires 36 are twisted as described above. The bias signal is synchronous with the electrical signal (e.g. , a pulsed 100-kHz sine wave signal) applied to the heating element 30. The bias signal, if not eliminated, creates a significant flow differential in the measurement of cardiac output. Flow differential is the difference in the measured flow of blood and the actual flow of blood. A bias signal decreases the integrity of the measurement. For example, if the actual flow of blood is 9 liters per minute (L/min) and the measured flow of blood is 10.3 L/min, then the flow differential is 1.3 L/min.

The inventors of the present invention have determined that conventional catheters having a standard configuration of substantially straight and parallel temperature-sensor wires typically yield flow differentials of up to or about 1.0 liter per minute (L/min). Many conventional catheters may yield flow differentials of greater than 1.5 L/min. If the actual blood flow is 9 L/min, then a flow differential of 1.5 L/min represents a nearly 17% error in measurement.

In contrast to conventional catheters, preferred embodiments of the reduced-noise catheter 20 of the present invention yield flow differentials of less than 0.5 L/min, with a number of the preferred embodiments of the catheter 20 yielding flow differentials of less than 0.25 L/min. Indeed, in testing performed by the inventors of the present invention, on average the reduced-noise catheter 20 may reduce the error in measuring blood flow by approximately 80%. Not only is this error reduction significant but it represents a critical and substantial advancement in the art of catheters. A plot of flow differentials of conventional catheters and of the reduced-noised catheter 20 of the present invention is illustrated in FIGURE 9. In addition, the inventors have determined that while the twisted temperature-sensor wires 36 significantly reduces noise of the signal reaching the processor 70, the twisted wires 36 have no direct effect on the DC voltage applied to the temperature sensor 32 or on the low voltage DC measurement of the temperature sensor 32 itself.

It has been determined that the error is caused by either thermal interference or electromagnetic interference, or a combination of both. Both the thermal and the electromagnetic interference may be caused by the activation of the heating element 30. The electrical signal applied to the heating element 30 by the control unit 68 is relatively large (e.g., about 15 watts) when compared to the electrical signal received by the processing unit 70 from the temperature sensor 32 (e.g., on the order of millivolts). Further, it has been determined that the errant DC bias voltage across the temperature sensor 32 is synchronous with the pulsing signal applied to the heating element 30. Accordingly, two time constants may be present: one resulting from thermal interference and one resulting from electromagnetic interference.

With further reference to FIGURE 1 and as mentioned above, the pitch of the temperature-sensor wires 36 preferably ranges from about two revolutions per cm to about six revolutions per cm. Although the temperature-sensor wires 36 may be twisted along substantially the overall length l_{BP} of the body portion 22 (less the distance l₁ from the temperature sensor 32 to the distal end 24 of the body portion 22), the reduced-noise catheter 20 may be configured with the temperature-sensor wires 36 twisted only along that section of the body portion 22 at which the heating element 30 is located. Thus, the temperature-sensor wires 35 may be twisted along length l_{HE} of the body portion 22, which length may be thought of as the zone of cross-talk. Alternatively, the temperature-sensor wires 36 may also be twisted along the length of the body portion 22 defined by the overall length l_{BP} less the distance l₂, i.e., the distance from the distal end of the heating element 30 to the distal end 24 of the body portion 22. This length of the body portion 22 represents the length along which the heating-element wires 34 and the temperature-sensor wires 36 are coexistent.

Regarding the size of the temperature-sensor wires 36, as the electrical signal carried by the temperature-sensor wires 36 to the processing unit 70 is relatively small in magnitude, the wires themselves may be relatively small (for example, less than about 0.25 mm (0.01 inch) in diameter) when compared to the heating-element wires 34. If the temperature sensor 32 includes a thermistor, the signal carried by the temperature-sensor wires 36 may be on the order of millivolts or smaller. Alternatively, if the temperature sensor 32 includes a current-based sensor, then the signal carried by the temperature-sensor wires 36 will be a small-magnitude current signal.

Because of the small size and because of the noise-eliminating twisted configuration, the temperature-sensor wires 36 may be received in the same lumen 28 as that of the heating wires 34, which embodiment is shown in FIGURE 2B. Alternatively, the diameter Ø_{TW} (See FIGURE 2A) of the lumen 28 in which the temperature-sensor wires 36 are to be received may be made smaller, thereby saving space in the body portion 22 or allowing the overall diameter Ø_{BP} (see FIGURE 1) of the body portion 22 to be accordingly smaller.

With reference to FIGURES 10A, 10B, and 10C, there are a number of commercially available processing units which may be used to process the electrical signals from the temperature sensor 32 to calculate blood temperature, blood flow, cardiac output, etc. One type of processing unit (for example, Supermodule™, which is available from Baxter Healthcare Corporation) used a voltage divider at the "front end" of the unit and applies a small voltage to the temperature sensor 32, which is schematically represented in FIGURE 10B. Another type of processing unit (for example, Vigilance®, which is also available from Baxter Healthcare Corporation) uses a current divider at the "front end" of the unit and applies a small current to the temperature sensor 32, which is schematically represented in FIGURE 10C. Accordingly, these two processing units 70a and 70b respond differently (i.e., oppositely) to the errant bias signal present on untwisted temperature-sensor wires 36. This different response is taken into consideration in configuring the preferred embodiment of the reduced-noise catheter 20 of the present invention.

With additional reference to FIGURE 11, taking into consideration the preferred embodiment in which the temperature sensor 32 is a thermistor, as temperature increases, which is indicated by ΔT, the resistance of the thermistor 32 decreases, as indicated by ΔR_{T}. Accordingly, the voltage measured by the voltage divider increases, as indicated by ΔV_{V}, whereas the voltage measured by the current divider decreases, as indicated by ΔV_{C}. The errant bias offset signal E will be additive in the voltage divider (in FIGURE 10B) and will be subtractive in the current divider (in FIGURE 10C). Accordingly, any changes in temperature or in true resistance of the thermistor 32 will cause a proportional but opposite change in the measured values of the processing units 70b and 70c. For example, if the actual blood flow is 9 L/min, then the voltage-divider processing unit 70b may measure 11 L/min, and the current-divider processing unit 70c may measure 7 L/min. This inconsistency in processing units 70 is eliminated by the reduced-noise catheter 20 of the present invention because the errant bias signal E is substantially eliminated. Thus, regardless of the type of processing unit 70 used in conjunction with the reduce-noise catheter 20, an accurate and confident measurement of the cardiac output may be calculated.

In a commercial embodiment the reduced-noise catheter 20, the overall length l_{BP} of the body portion 22 may be any desired length suitable for measuring cardiac output in a patient, which is preferably about 110 cm, thereby allowing the body portion 22 of the catheter 20 to be inserted into the patient femorally. Further, the distance 1, from the thermistor 32 to the distal end 24 of the body portion 22 may range up to about 10 cm but preferably ranges from about 3 cm to about 5 cm. The distance between the distal end of the heating element 32 (which may be activated either a DC signal or an AC signal) and the temperature sensor 30 (i.e., l₂ minus l₁) may range up to about 20 cm. The outer diameter Ø_{BP} of the body portion 22 is preferably about 2.33 mm (7 French), although this diameter depends upon the number of lumens 28 of the body portion 22, the size of the lumens 28, and so on. In addition, the outer surface of the body portion 22 may have distance marks 72 evenly spaced along the length which indicate to a physician the distance the body portion 22 has been inserted into the vascular system of a patient.

The reduced-noise catheter 20 preferably has about 5 to 7 lumens 28. The lumens 28, other than those used to house the heating-element and temperature-sensor wires 34 and 36, may be used for any purpose known in the art, for example, as an inflation lumen in conjunction with a balloon (not shown) disposed at the distal end 24 of the body portion 22. Catheter model Nos. 139, 746, and 757 available from the Edwards Critical Care of Irvine, California, which is a division of Baxter International Incorporated of Deerfield, Illinois, are examples of multi-lumen catheters which may be used for the reduced-noise catheter of the present invention. Further, the catheter 20 may be configured with any suitable commercially available temperature sensor or thermistor.

## Claims

1. A reduced-noise catheter (20) comprising:
a) a body portion (22) having a distal end (24) and a proximal end (26);
b) a plurality of lumens (28) formed in the body portion between the distal end and the proximal end;
c) a heating element (30) disposed between the distal end and the proximal end of the body portion;
d) a temperature sensor (32) disposed between the heating element and the distal end of the body portion;
e) heating-element wires (34) connected to the heating element, extending from the heating element to the proximal end of the body portion in one of the lumens for communicating with a control unit, and for carrying an activation signal from the control unit to the heating element to activate the heating element; and
f) temperature-sensor wires (36) connected to the temperature sensor, extending in one of the lumens for communicating with a processing unit, and for carrying a temperature-sensor signal from the temperature sensor to the processing unit for processing, **characterized by** said temperature-sensor wires extending in a twisted configuration from the temperature sensor to the proximal end of the body portion.

2. The reduced-noise catheter of claim 1 wherein the temperature-sensor wires are twisted bifilar wires attached along a seam.

3. The reduced-noise catheter of claim 1 or claim 2 wherein the temperature-sensor wires are separate wires twisted together.

4. The reduced-noise catheter according to any of the preceding claims wherein the heating-element wires and the temperature-sensor wires extend to the proximal end in the same lumen.

5. The reduced-noise catheter according to any of the preceding claims wherein the temperature-sensor wires have a pitch of at least two revolutions per cm.

6. The reduced-noise catheter according to any of the preceding claims wherein the temperature sensor includes a thermistor.

7. The reduced-noise catheter according to any of the preceding claims, wherein the temperature sensor has a portion exposed to the exterior of the body, the temperature sensor having a variable electrical resistance dependent on the temperature of the exposed portion.

8. The reduced-noise catheter according to any of the preceding claims, wherein the catheter includes a current divider to determine the resistance of the temperature sensor.

9. The reduced-noise catheter according to any of the preceding claims, wherein the catheter includes a voltage divider to determine the resistance of the temperature sensor.

10. A method of reducing noise in a catheter, the catheter including a temperature sensor, a heating element, and a plurality of lumens, the noise being reduced in a signal from the temperature sensor, the method comprising the step of:
twisting temperature-sensor wires connected to the temperature sensor at least along a portion of the catheter at which the heating element is disposed, the temperature-sensor wires being received within one of the lumens of the catheter.

11. The method according to claim 10 wherein the temperature-sensor wires are twisted along the entire length of the lumen within which the temperature-sensor wires are received.

12. The method according to claim 10 or claim 11 further comprising the step of:
positioning the temperature-sensor wires in the same lumen within which heating-element wires connected to the heating element are received.

13. The method according to any of claims 10 to 12 wherein the temperature sensor is located on a distal end of the catheter and has a variable resistance to temperature, the temperature sensor wires extending to a proximal end of the catheter, the heating element being disposed between the distal and proximal ends of the catheter and including electrically conductive portions exposed to the exterior of the catheter body and surrounding the temperature wires, and wherein the method further comprises transmitting a signal through the temperature sensor, reducing noise in the signal associated with the electromagnetic field of the heater element by twisting the temperature sensor wires in at least a portion surrounded by the heater element, and processing the reduced noise signal transmitted through the temperature sensor to determine the resistance of the temperature sensor.

14. The method of claim 13, wherein the step of processing includes using a current divider to determine the resistance of the temperature sensor.

15. The method of claim 13 or claim 14, wherein the step of processing includes using a voltage divider to determine the resistance of the temperature sensor.

## Patentansprüche

1. Rauschreduzlerter Katheter (20) mit
a) einem Korperberelch (22) mit einem distalen Ende (24) und einem proximalen Ende (25);
b) mehreren Lumen (28), die in dem Körperbereich zwischen dem distalen Ende und dem proximalen Ende gebildet sind;
o) einem Helzelement (30), welches zwischen dem distalen Ende und dem proximalen Ende des Körperbereiches angeordnet ist;
d) einem Temperatursensor (32), der zwischen dem Helzelement und dem distalen Ende des Körperbereiches angeordnet ist;
e) Helzelement-Kabeln (34), die mit dem Helzelement verbunden sind und sich von dem Heizelement zu dem proximalen Ende des Körparbersiches In einem der Lumen erstrecken für eine Kommunlkation mit einer Stauerungs- oder Regeleinheit und für eine Oberrmlttlung eines Aktivierungssignals von der Steuerungs- oder Regeleinheit zu dem Heizelement, um das Heizelement zu aktivieren; und
f) Temperatursensor-Kabeln (36), die mit dem Temperatursensor verbunden sind und sich in eines der Lumen erstrecken für eine Kommunikation mit einer Verarbeltungseinheit und for eine Übermittlung eines Temperatursensor-Signals von dem Temperatursensor zu der Verarbeitungseinheit für ein Verarbeiten, **dadurch gekennzeichnet, dass** sich die Temperatursensor-Kabel in verdrehter oder verflochtener Konfiguration von dem Temperatursensor zu dem proximalen Ende des Körperbereiches erstrecken.

2. Katheter nach Anspruch 1, **dadurch gekennzeichnet, dass** die Temperatursensor-Kabel zweiedrige verdrehte oder verflochtene Kabel sind, die entlang eines Saumes aneinander befestigt sind.

3. Katheter nach Anspruch 1 oder 2, **dadurch gekennzeichnet, dass** die Temperatursensor-Kabel separate Kabel sind, die zusammengedreht oder zusammengeflochten sind.

4. Katheter nach einem der vorhergehenden Ansprüche, **dadurch gekennzeichnet, dass** die Heizelement-Kabel und die Temperatursensor-Kabel sich zu dem proximalen Ende In demselben Lumen erstrecken.

5. Katheter nach einem der vorhergehenden Ansprüche, **dadurch gekennzeichnet, dass** die Temperatursensor-Kabel eine Steigung besitzen, so dass sich zumindest zwei Umdrehungen pro Zentimeter ergeben.

6. Katheter nach einem der vorhergehenden Ansprüche, **dadurch gekennzeichnet, dass** der Temperatursensor einen Ternporsturfühler besitzt.

7. Katheter nach einem der vorhergehenden Ansprüche, **dadurch gekennzeichnet, dass** der Temperatursensor einen Bereich hat. der mit dem Außeren des Körpers in Wirkverbindung sieht wobei der Temperatursensor einen variablsn elektrischen Widerstand in Abhängigkeit von der Temperatur des dem Äußern auageestzten Bereiches besitzt.

8. Katheter nach einem der vorhergehenden Ansprüche, **dadurch gekennzeichnet, dass** der Katheter einen Stromteller besitzt, um den Widerstand das Temperatursensors zu ermitteln.

9. Katheter nach einem der vorhergehenden Ansprüche, **dadurch gekennzeichnet, dass** der Katheter einen Spannungsteiler besitzt, um den Widerstand des Tempenabu rsensors zu ermitteln.

10. Verfahren zur Reduzierung das Rauschens In einem Katheter, wobei der Katheter einen Temperatursensor, ein Heizelement und mehrere Lumen besitzt und das Rauschen In einem Signal von dem Temperatursensor reduziert wird, mit folgendem Verfahrensschritt:
Verdrehen oder Verflechten von Temperatursensor-Kabein, die mit dem Temperatursensor verbunden sind, zumindest In einem Bereich des Katheters, In dem das Heizelement angeordnet Ist, wobei die
Temperatursensor-Kabel in einem der Lumen des Katheters aufgenommen sind.

11. Verfahren nach Anspruch 10, **dadurch gekennzeichnet, dass** die Temperatursensor-Kabel entlang der gesamten Länge des Lumens, In dem die Temperatursensor-Kabel aufgenommen werden, verdraht oder verflochten werden.

12. Verfahren nach Anspruch 10 oder 11, wobei die Temperatursensor-Kabel In demselben Lumen positioniert worden, In dem mit dem Heizelement verbundene Heizelement-Kabel aufgenommen sind.

13. Verfahren nach einem der Ansprüche 10 bis 12, **dadurch gekennzeichnet, dass** der Temperatursensor an einem distalen Ende des Katheters angeordnet ist und einen hinsichtlich Temperatur variablen Widerstand besitzt, sich die Temperatursensor-Kabel zu dem proximalen Ende des Katheters erstrecken, das Heizelement zwlschen dem distalen und proximalen Ende des Katheters angeordnet ist und elektrisch konduktlve Bereiche besitzt, die dem Äußeren des Katheterkörpers ausgesetzt sind und die Temperaturkabel umgeben, wobei durch den Temperatursensor ein Signal übertragen wird und Rauschen in dem Signal In Verbindung mit dem elektromagnetischen Feld des Heizelementes durch Verdrehen oder Verflechten der Temperatursensor-Kabel in zumindest einem Bereich, der durch das Heizelement umgeben ist, reduziert wird und das Signal mit reduziertem Rauschen, das durch den Temperatursensor zur Bestimmung des Widerstandes des Temperatursensors übertragen wird, verarbeitet wird.

14. Verfahren nach Anspruch 13, **dadurch gekennzeichnet, dass** das Verarbeiten eine Verwendung eines Stromteilers beinhaltet um den Widerstand des Temperatursensors zu ermitteln.

15. Verfahren nach Anspruch 13 oder Anspruch 14, **dadurch gekennzeichnet, dass** das Verarbeiten eine Verwendung eines Spannungstellers beinhaltet, um den Widerstand des Temperatursensors zu ermitteln.

## Revendications

1. Cathéter (20) à bruit réduit comprenant :
a) une partie de corps (22) ayant une extrémité distale (24) et une extrémité proximale (26) ;
b) une pluralité de lumières (28) formées dans la partie de corps entre l'extrémité distale et l'extrémité proximale;
c) un élément chauffant (30) placé entre l'extrémité distale et l'extrémité proximale de la partie de corps ;
d) un capteur de température (32) placé entre l'élément chauffant et l'extrémité distale de la partie de corps ;
e) des fils d'élément chauffant (34) raccordés à l'élément chauffant, s'étendant de l'élément chauffant à l'extrémité proximale de la partie de corps dans l'une des lumières, pour communiquer avec une unité de commande et pour porter un signal d'activation provenant de l'unité de commande vers l'élément chauffant pour activer l'élément chauffant ; et
f) des fils de capteur de température (36) raccordés au capteur de température, s'étendant dans l'une des lumières pour communiquer avec une unité de traitement et pour porter un signal de capteur de température provenant du capteur de température vers l'unité de traitement pour traitement, **caractérisé par le fait que** lesdits fils de capteur de température s'étendent en une configuration enroulée depuis le capteur de température vers l'extrémité proximale de la partie de corps.

2. Cathéter à bruit réduit de la revendication 1 dans lequel les fils de capteur de température sont des fils bifilaires enroulés fixés le long d'une couture.

3. Cathéter à bruit réduit de la revendication 1 ou de la revendication 2 dans lequel les fils de capteur de température sont des fils séparés enroulés ensemble.

4. Cathéter à bruit réduit selon l'une quelconque des revendications précédentes dans lequel les fils d'élément chauffant et les fils de capteur de température s'étendent vers l'extrémité proximale dans la même lumière.

5. Cathéter à bruit réduit selon l'une quelconque des revendications précédentes, dans lequel les fils de capteur de température ont un pas d'au moins deux tours par cm.

6. Cathéter à bruit réduit selon l'une quelconque des revendications précédentes dans lequel le capteur de température inclut une thermistance.

7. Cathéter à bruit réduit selon l'une quelconque des revendications précédentes dans lequel le capteur de température a une partie exposée à l'extérieur du corps, le capteur de température ayant une résistance électrique variable dépendant de la température de la partie exposée.

8. Cathéter à bruit réduit selon l'une quelconque des revendications précédentes dans lequel le cathéter inclut un diviseur de courant pour déterminer la résistance du capteur de température.

9. Cathéter à bruit réduit selon l'une quelconque des revendications précédentes dans lequel le cathéter inclut un diviseur de tension pour déterminer la résistance du capteur de température.

10. Procédé pour réduire le bruit dans un cathéter, le cathéter incluant un capteur de température, un élément chauffant et une pluralité de lumières, le bruit étant réduit en un signal provenant du capteur de température, le procédé comprenant une étape :
d'enroulement des fils de capteur de température raccordés au capteur de température au moins le long d'une partie du cathéter au niveau de laquelle l'élément chauffant est placé, les fils de capteur de température étant reçus dans l'une des lumières du cathéter.

11. Procédé selon la revendication 10 dans lequel les fils de capteur de température sont enroulés sur toute la longueur de la lumière dans laquelle les fils de capteur de température sont reçus.

12. Procédé selon la revendication 10 ou la revendication 11 comprenant en outre une étape :
de positionnement des fils de capteur de température dans la même lumière dans laquelle les fils d'élément chauffant raccordés à l'élément de chauffage sont reçus.

13. Procédé selon l'une quelconque des revendications 10 à 12 dans lequel le capteur de température est situé sur une extrémité distale du cathéter et a une résistance variable à la température, les fils de capteur de température s'étendant vers une extrémité proximale du cathéter, l'élément chauffant étant placé entre les extrémités distale et proximale du cathéter et incluant des parties électriquement conductrices exposées à l'extérieur du corps du cathéter et entourant les fils de température, et dans lequel le procédé comprend en outre la transmission d'un signal à travers le capteur de température, la réduction du bruit dans le signal associé au champ électromagnétique de l'élément chauffant en enroulant les fils de capteur de température dans au moins une partie entourée par l'élément chauffant, et le traitement du signal de bruit réduit à travers le capteur de température pour déterminer la résistance du capteur de température.

14. Procédé de la revendication 13, dans lequel l'étape de traitement inclut l'utilisation d'un diviseur de courant pour déterminer la résistance du capteur de température.

15. Procédé de la revendication 13 ou de la revendication 14, dans lequel l'étape de traitement inclut l'utilisation d'un diviseur de tension pour déterminer la résistance du capteur de température.
